# EUROPÄISCHE PATENTSCHRIFT

(11) **EP 3 068 487 B1**
(45) Veröffentlichungstag und Bekanntmachung des Hinweises auf die Patenterteilung: **18.12.2019**
(21) Anmeldenummer: 14801972.2
(22) Anmeldetag: 11.11.2014
(51) Int. Cl.: A61N 5/06

(54) **RAUM UND LICHTDECKENSYSTEM ZUR DURCHFÜHRUNG DER PHOTODYNAMISCHEN THERAPIE**
SPACE AND LUMINOUS CEILING SYSTEM FOR CONDUCTING PHOTODYNAMIC THERAPY
PIÈCE ET SYSTÈME DE PLAFOND LUMINEUX DE MISE EN UVRE DE LA THÉRAPIE PHOTODYNAMIQUE

(30) Priorität: 13.11.2013 DE 202013105127 U
(43) Veröffentlichungstag der Anmeldung: 21.09.2016
(73) Patentinhaber: Swiss Red AG, 3280 Murten (CH)
(72) Erfinder: DIETZ, John, 53175 Bonn (DE); REINHOLD, Uwe, 53125 Bonn (DE)
(74) Vertreter: Wagner Albiger & Partner Patentanwälte mbB
(86) Internationale Anmeldenummer: PCT/EP2014/074275
(87) Internationale Veröffentlichungsnummer: WO 2015/071262

(56) Entgegenhaltungen:
- EP-A2- 1 147 785
- WO-A1-92/20211
- WO-A2-2004/080291
- DE-A1- 2 313 103
- DE-U1-202013 003 573
- BOZJA J ET AL: "Porphyrin-Based, Light-Activated Antimicrobial Materials", JOURNAL OF POLYMER SCIENCE PART A: POLYMER CHEMISTRY, JOHN WILEY & SONS, INC, US, Bd. 41, 1. Januar 2003 (2003-01-01), Seiten 2297-2303, XP002535483, ISSN: 0887-624X, DOI: 10.1002/POLA.10773
- BHASIN H KAUSAR M ATHAR G: "Ferrochelatase, a novel target for photodynamic therapy of cancer", ONCOLOGY REPORTS, SPANDIDOS PUBLICATIONS, GR, Bd. 6, Nr. 6, 1. November 1999 (1999-11-01), Seiten 1439-1442, XP009182053, ISSN: 1021-335X, DOI: 10.3892/OR.6.6.1439

## Beschreibung

Die Erfindung betrifft einen Raum zur Durchführung der Photodynamischen Therapie (PDT).

Die Photodynamische Therapie (PDT) hat die sauerstoffabhängige Zerstörung von Tumorzellen der Haut zum Ziel, die nach einer vorangegangenen Photosensibilisierung und Belichtung geeigneter Wellenlänge eintritt. Die Zerstörung erfolgt selektiv, wobei im Rahmen der PDT Tumorzellen und deren Vorstufen, nicht aber die gesunden Zellen zerstört werden.

Bei der PDT wird auf das Hautareal mit krankem Gewebe eine Creme mit zum Beispiel dem Wirkstoff 5-alpha-Aminolävulinsäure (ALA) als Photosensibilisator zur Bildung lichtempfindlicher Porphyrine, insbesondere Protoporphyrin 9 (PPIX), aufgetragen. Der Wirkstoff ALA und somit die daraus sich bildenden Porphyrine reichern sich in entzündlich verändertem Gewebe und bösartigen Tumoren intensiv an. Dadurch wird eine selektive Behandlung und Zerstörung krankhaft veränderter Hautstrukturen ermöglicht. Durch die Bestrahlung nehmen die Porphyrine Energie auf und übertragen diese auf den Sauerstoff in den Zellen. Durch die Energieaufnahme geht der "normale" Triplettsauerstoff in einen angeregten Zustand über (Singulettsauerstoff). Diese Sauerstoffradikale zerstören die betroffenen Zellen, wobei durch die lokale Wirkung der Freisetzung der Sauerstoffradikalen eine weitergehende Schädigung von gesunden Zellen ausbleibt. Beispielsweise wird in der Publikation von Bhasin et al ("Ferrochelatase, a novel target for photodynamic therapy of cancer", ONCOLOGY REPORTS, SPANDIDOS PUBLICATIONS, GR, Bd. 6, Nr. 6, 1. November 1999 (1999-11-01), Seiten 1439-1442, XP009182053, ISSN: 1021-335X, DOI: 10.3892/OR.6.6.1439"). der Einsatz von PPIX zur Bekämpfung von Hautkrebs beschrieben. Aus Bozja et al ("Porphyrin-Based, Lights-Activated Antimicrobial Materials", JOURNAL OF POLYMER SCIENCE PART A: POLYMER CHEMISTRY, JOHN WILEY & SONS, INC, US, Bd. 41, 1. Januar 2003 (2003-01-01), Seiten 2297-2303, XP002535483, ISSN: 0887-624X, DOI: 10.1002/POLA.10773") geht hervor, mit PPIX Textilfasern zu veredeln, um antimikrobielles Gewebe für Arztkittel und dgl. herzustellen.

Grundsätzlich kann zwischen der geschlossenen PDT und der offenen Tageslicht-PDT unterschieden werden. Bei der geschlossenen PDT wird das zu behandelnde Hautareal eingecremt und danach ein lichtundurchlässiger Verband (zum Beispiel Alufolie) appliziert. Nach drei- bis vierstündiger Einwirkzeit wird der Verband entfernt. Der Wirkstoff hat sich in dieser Zeit in den kranken Zellen angereichert, während die gesunden Zellen diesen kaum aufnehmen. In den kranken Zellen bilden sich die photoaktiven Porphyrine. Das betroffene Areal wird dann etwa 10 Minuten mit einer speziellen PDT-Lampe intensiv bestrahlt. Die Beleuchtungsintensität ist dabei sehr hoch und führt trotz der relativ kleinen Beleuchtungszeiten zu einer Lichtdosis von bis zu 200 J/cm². Bei PDT-Lampen mit einem schmaleren Lichtspektrum lässt sich die erforderliche Lichtdosis bei gleichem therapeutischen Effekt deutlich reduzieren, doch auch hier liegen die Werte für die Lichtdosis bei rund 37 J/m².

Die Bestrahlung durch die PDT-Lampe bei der geschlossenen PDT kann zum Teil zu starken Schmerzen führen. Im Zusammenhang mit der geschlossenen PDT ist es daher bekannt, vor der Bestrahlung Schmerzmittel zu verabreichen. Beispielsweise lässt sich mit einer Bestrahlungsvorrichtung der DE 10 2013 003 573 U1 eine solche Bestrahlung durchführen.

Im Gegensatz zu der geschlossenen PDT gilt die so genannte Tageslicht-PDT als schmerzfrei. Bei der Tageslicht-PDT wird ebenfalls der Wirkstoff ALA aufgetragen, allerdings wird nur ca. 30 Minuten gewartet und dann über einen Zeitraum von 1,5 - 3 Stunden das eingecremte Hautareal unter Ausnutzung des natürlichen Tageslichts belichtet. Bei der Tageslicht-PDT reicht eine Bestrahlungsenergie von 10 J/cm² aus.

Bei der Tageslicht-PDT hängt die Belichtungsleistung oder Belichtungsstärke von dem geographischen Ort, der geodätischer Höhe, dem Sonnenstand, der Jahreszeit und dem augenblicklichen Wetter ab. So kann es beispielsweise sein, dass bei ungünstigem Wetter während der Winterzeit mehrere Wochen die Tageslicht-PDT nicht durchgeführt werden kann, da das Tageslicht zu schwach ist, die Porphyrine ausreichend anzuregen, um Singulettsauerstoff zur Zerstörung der Tumorzellen entstehen zu lassen. So geht die Beleuchtungsstärke an einem trüben Wintertag nicht über 4000 Lux hinaus.

Der Erfindung liegt daher die Aufgabe zu Grunde, eine Vorrichtung bereitzustellen, mit deren Hilfe die Photodynamische Therapie zur Behandlung von Hautkrankheiten durchgeführt werden kann, wobei die PDT möglichst schmerzfrei und ohne zeitliche Restriktionen jederzeit durchführbar sein soll.

Die der Erfindung zu Grunde liegende Aufgabe wird mit dem Anspruch 1 gelöst. Ausführungsbeispiele der Erfindung können den übrigen Ansprüchen entnommen werden. Die Erfindung ist in den Ansprüchen definiert; andere Ausführungen sind nur illustrativ.

Der erfindungsgemäße Raum dient der Durchführung der PDT, durch die Hautkrankheiten behandelt werden können, wobei hier die Behandlung von oberflächigen Hauttumoren im Vordergrund steht. Als Beispiele für derartige Hauttumore können aktinische Keratosen, flache Basaliome und initiale Karzinome genannt werden.

Der erfindungsgemäße Raum weist Seitenwände, eine Decke, einen Boden und wenigstens zwei Leuchten auf, die mindestens 50 Zentimeter voneinander beabstandet sind. Erfindungsgemäß leuchten die wenigstens zwei Leuchten einen Teilbereich des Raumes aus, wobei an jedem Ort im Teilbereich eine Beleuchtungsstärke von mindestens 8000 Lux vorliegt und der Teilbereich eine horizontale Ausdehnung von mindestens einem Quadratmeter (1 m²) und eine vertikale Höhe von mindestens von 40 Zentimeter (40 cm) aufweist.

In einem Ausführungsbeispiel beträgt die minimale Beleuchtungsstärke an jedem Ort im Teilbereich 10.000, 12.000 oder gar 15.000 Lux. Die horizontale Ausdehnung (maximale Flächenausdehnung des Teilbereichs in einer horizontalen Ebene) kann größer als 2, 4 oder 8 Quadratmeter sein. Die vertikale Höhe des Teilbereichs (maximale vertikale Höhe) kann bis zu 50, 70 oder 100 Zentimeter betragen. Der Abstand zwischen zwei Leuchten kann auch größer als 70 oder 100 Zentimeter sein, wobei es auf den Abstand zweier beliebiger Leuchten und nicht zwangsläufig auf den Abstand benachbarter Leuchten ankommt. Ein Mindestabstand benachbarter Leuchten kann 20, 30 oder 40 Zentimeter betragen.

In einer Ausführung der Erfindung nimmt die Beleuchtungsstärke innerhalb des Teilbereichs nur Werte an, die kleiner sind als 30.000 Lux. Die maximale Beleuchtungsstärke kann auch nur 25.000 oder gar 20.000 Lux betragen.

Wird ein Patient nach erfolgter Applizierung des Photosensibilisators auf das zu bestrahlende Hautareal in den erfindungsgemäßen Raum gesetzt oder gelegt, so dass sich das betreffende Hautareal innerhalb des Teilbereichs des Raumes befindet, und für ca. 1,5 - 3 Stunden mit dem Licht der wenigstens zwei Leuchten bestrahlt, so lassen sich gleiche therapeutische Effekte wie bei der klassischen geschlossenen PDT erzielen. Jedoch besteht ein großer Unterschied darin, dass der Patient während der Bestrahlung in dem erfindungsgemäßen Raum kein Schmerz verspürt. Im Vergleich zu der Tageslicht-PDT besteht ein wesentlicher Unterschied darin, dass die PDT von nicht beeinflussbaren Parametern (zum Beispiel Wetter, Jahreszeit) völlig unabhängig ist. Vielmehr lässt sich die Beleuchtungsstärke bzw. die Beleuchtungsintensität zielgenau einstellen, und die PDT lässt sich jederzeit an jedem Ort unter gleichen Randbedingungen durchführen. Mit einer derartigen PDT lässt sich eine Lichtdosis von mindestens 10 J/cm² erreichen.

Auch wird es als vorteilhaft erachtet, dass sich der Patient während der Bestrahlungszeit im Raum frei bewegen kann, solange sich das zu behandelnde Hautareal in dem Teilbereich befindet und dem Licht der Leuchten ausgesetzt ist.

Erfindungsgemäß weicht innerhalb des Teilbereichs die Beleuchtungsstärke nicht mehr als 50 Prozent vom Maximalwert im Teilbereich ab. Wird beispielsweise im Teilbereich an einem Ort ein Maximalwert von 22.000 Lux erreicht, so sind mindestens an jedem Ort des Teilbereichs 11.000 Lux gefordert. Somit herrscht innerhalb des gesamten Teilbereichs eine Bestrahlungsintensität auf etwa gleichem Niveau, dass einerseits nicht zu Schmerzen führt und andererseits aber auch im ausreichenden Maße über die angeregten Porphyrine die Bildung von Singulettsauerstoff ermöglicht.

Vorzugsweise sind die Leuchten an der Decke oder in einer Zwischendecke des Raumes fest installiert. Wie auch bei der Tageslicht-PDT erfolgt die Strahlung dann in erster Linie von oben. Alternativ oder zusätzlich können die Leuchten auch an der Seitenwand und am Boden installiert werden. Optimale Ergebnise werden aber durch deckennahe Leuchten erzielt. Die Seitenwände und der Boden können als reflektierende Flächen eingesetzt werden, was die Beleuchtungsstärke im Teilbereich verstärken kann. Der Teilbereich kann innerhalb des Raumes außermittig angeordnet werden, um so beispielsweise die Reflektion der Seitenwände in einer Ecke des Raums besser ausnutzen zu können.

Ein Abstand zwischen dem Boden und der Zwischendecke des Raumes beträgt zwischen 230 und 350 Zentimeter. Somit würde der erfindungsgemäße Raum eine Zimmerhöhe aufweisen, die der üblichen Zimmerhöhe in alten oder neuen Gebäuden entspricht. Es wäre somit mit geringem Aufwand möglich, vorhandene Behandlungszimmer in einer dermatologischen Praxis zu einem erfindungsgemäßen Raum umzubauen.

Ein Abstand zwischen dem Boden und dem Teilbereich beträgt 40 bis 120 Zentimeter (bezogen auf ein unteres Ende des Teilbereichs). Geht man beispielsweise von einer vertikalen Höhe des Teilbereichs von 50 Zentimeter, einem Abstand zwischen Boden und Teilbereich von einem Meter und einer Deckenhöhe von 250 Zentimeter aus, so würde sich ein Abstand zwischen den Leuchten und dem Teilbereich (oberes Ende des Teilbereichs) von einem Meter ergeben.

Die Leuchten weisen jeweils ein Leuchtmittel auf, das im Wellenlängenbereich von 560 bis 660 nm kontinuierlich Licht emittiert. D.h., dass das Lichtspektrum dieses Leuchtmittels in diesem Wellenlängenbereich keine Lücken aufweist. Insbesondere wird ein Licht mit einer Wellenlänge von 630 nm (Nanometer) emittiert, dessen Energie die Porphyrine aufnehmen. Das Leuchtmittel ist darüber hinaus so beschaffen, dass es in diesem Bereich mehr als 75 Prozent (vorzugsweise mehr als 80 oder 90 Prozent) seiner Gesamtstrahlung abgibt. Somit ist die Strahlungsdichte des Leuchtmittels für Wellenlängen kleiner 560 nm und größer 660 nm verhältnismäßig klein. Ein anderes bevorzugtes Leuchtmittel emittiert mindestens 80 % (vorzugsweise mehr als 90 %) seiner Strahlungsenergie in einem Wellenlängenbereich von 600 bis 650 nm.

Die Leuchten können in zwei oder mehr parallelen Reihen angeordnet sein. Beispielsweise ist eine Leuchtenanordnung denkbar, die aus insgesamt acht Leuchten besteht, die in zwei Viererreihen angeordnet sind. Es ist auch möglich, eine zentral angeordnete Leuchte vorzusehen, um die in einem gleichen Abstand auf einem konzentrischen Kreis mehrere Außenleuchten angeordnet sind.

Jede Leuchte kann einen Reflektor aufweisen, der das Licht bündelt und/oder in bestimmter Weise in eine Richtung leitet, so dass sich das Licht mehrerer Leuchten so überlagert, dass der Teilbereich möglichst gut (zum Beispiel möglichst gleichmäßig in einer horizontalen Ebene) ausgeleuchtet wird. Jede Leuchte kann einen symmetrischen oder asymmetrischen Reflektor aufweisen.

Die Ausrichtung eines asymmetrischen Reflektors kann dabei so erfolgen, dass bei einer Leuchtenanordnung mit mehreren Leuchten sich die asymmetrischen Reflektoren zu einem symmetrischen Gesamtgebilde einfügen. Sollten an der Decke Hindernisse (zum Beispiel Lüftungsrohre) vorhanden sein, so kann durch Auswahl und individuelle Auslegung der Reflektoren ein im Wesentlichen homogen ausgeleuchteter Teilbereich erzielt werden, selbst wenn die Leuchten an der Decke nicht in einem gleichmäßigen Muster installiert sind.

Die Leuchten sind bündig in einer Zwischendecke eingebaut. Dies bedeutet, dass eine Unterkante eines Reflektor und die Zwischendecke im Wesentlichen in einer gemeinsamen Ebene liegen. Um einer zu starken Temperaturerhöhung in dem Zwischenraum zwischen Zwischendecke und Decke entgegenzuwirken, kann eine Kühlvorrichtung beispielsweise in Form eines Gebläses vorgesehen sein.

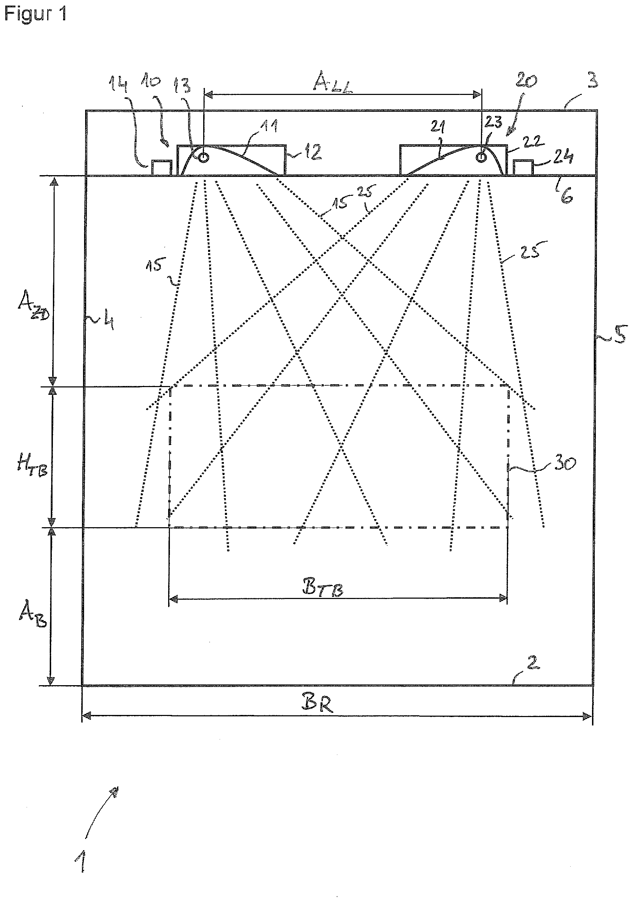

Anhand eines in der einzigen Figur 1 gezeigten Ausführungsbeispiels soll die Erfindung näher erläutert werden. Figur 1 zeigt im Querschnitt einen Raum, der in seiner Gesamtheit mit 1 bezeichnet wird. Der Raum 1 weist einen Boden 2, eine Decke 3 sowie Seitenwände auf, von denen jedoch nur eine linke Seitenwand 4 und eine rechte Seitenwand 5 in der Abbildung der Figur 1 zu erkennen sind.

In einem Abstand zur Decke 3 verläuft parallel dazu eine Zwischendecke 6. Der Abstand zwischen Deckel 3 und Zwischendecke 6 kann beispielsweise 20 - 30 Zentimeter betragen. In der Zwischendecke 6 sind eine erste Leuchte 10 und eine zweite Leuchte 20 fest montiert. Da die Leuchten 10, 20 baugleich ausgeführt sind und lediglich spiegelverkehrt eingebaut sind, wird im Folgenden Bezug genommen nur auf die erste Leuchte 10. Die folgenden Ausführungen zur ersten Leuchte 10 gelten somit sinngemäß auch für die zweite Leuchte 20.

Die erste Leuchte 10, die hier als Flächenstrahler ausgebildet ist, weist einen asymmetrischen Reflektor 11 auf, der in einem Leuchtengehäuse 12 untergebracht ist. Ein Leuchtmittel 13, das oberseitig von dem Reflektor 11 umschlossen wird, weist ein Lichtspektrum auf, dass im Bereich oder in der Nähe von 630 nm eine große Strahlungsdichte aufweist. Zum Starten und/oder Betrieb des Leuchtmittels 13 ist ein Vorschaltgerät 14 vorgesehen, das außerhalb des Leuchtengehäuses 12 angeordnet ist. Eine derartige Anordnung dient dazu, das Vorschaltgerät vor zu großer Wärme zu schützen, die in der ersten Leuchte 10 erzeugt wird. Die elektrische Leistung der Leuchte 10 kann 400 Watt und mehr (zum Beispiel 500 bis 1000 Watt) betragen.

Bei dem Leuchtmittel 13 handelt es sich um eine im Wesentlichen zylinderförmige Lampe mit einem Durchmesser von 2 bis 4 Zentimeter und einer Länge von 15 bis 25 Zentimeter. Somit erstrecken sich der asymmetrische Reflektor 11 und das Leuchtmittel mit dem hier gezeigten und im Wesentlichen konstanten Querschnitt in die Zeichenebene hinein. Das Leuchtengehäuse 12 weist somit die Form eines Quaders auf. Das Leuchtengehäuse kann eine quadratische Form aufweisen (d. h. von oben oder unten betrachtet ist der Querschnitt des Leuchtengehäuses quadratisch). Damit lässt sich die Leuchte 10 gut in vorhandene Zwischendecken integrieren, die üblicherweise ein quadratisches Raster aufweisen.

Die erste Leuchte 10 emittiert Licht, was durch die dargestellten Lichtstrahlen 15 verdeutlicht sein soll. Diese Strahlen 15 überlagern sich mit entsprechenden Lichtstrahlen 25 der zweiten Leuchte 20.

Innerhalb des Raumes 1 lässt sich ein Teilbereich definieren, der mit 30 bezeichnet ist. Der Teilbereich 30 weist eine vertikale Höhe H_{TB} und in horizontaler Richtung eine Breite B_{TB} auf. Ein Abstand zum Boden 2 ist mit A_{B} und ein Abstand zur Zwischendecke 6 ist mit A_{ZD} bezeichnet. Beträgt beispielsweise eine Breite des Raumes B_{R} 2,50 m, so ist im hier gezeigten Ausführungsbeispiel der Teilbereich 30 ungefähr 1,60 m breit (B_{TB} = 1,6 m). Die Höhe H_{TB} beträgt rund 70 cm, wobei der Abstand zum Boden A_{B} rund 80 cm beträgt. Der Abstand A_{ZD} zwischen Teilbereich 30 und der Zwischendecke 6 misst rund 100 cm. Ein Abstand A_{LL} zwischen den beiden Leuchten 10, 20 (gerechnet jeweils vom Mittelpunkt der Leuchtmittel 13, 23), beträgt rund 1,3 m.

Wie oben bereits ausgeführt, ist Figur 1 eine Querschnittsdarstellung. Der Teilbereich 30 erstreckt sich somit in die Zeichenebene hinein und kann beispielsweise eine Länge von 3 Meter aufweisen. In diesem Fall würde sich eine horizontale Ausdehnung von 4,80 m² ergeben (B_{TB} = 1,6 m; B_{TB} x 3 m). Innerhalb des Teilbereichs 30 ist eine Beleuchtungsstärke gegeben, die in jedem Ort des Teilbereichs mindestens größer als 12.000 Lux, aber kleiner als 30.000 Lux sein soll. Um auch in Längsrichtung des Teilbereiches (also senkrecht zur Zeichenebene) eine genügend große Beleuchtungsstärke sicherzustellen, sind, in der Darstellung der Figur 1 betrachtet, hinter der ersten Leuchte 10 drei weitere Leuchten angeordnet. Das gleiche gilt sinngemäß für die zweite Leuchte 20, so dass der Raum 1 von insgesamt acht Leuchten, die in zwei Reihen nebeneinander angeordnet sind, ausgeleuchtet wird. Dabei überlagert sich das Licht nicht nur von zwei sich gegenüberliegenden Leuchten (wie die in Figur 1 gezeigte erste und zweite Leuchte 10, 20), sondern auch von Leuchten in einer gleichen Reihe. Der Teilbereich 30, der hier im Querschnitt eine rechtwinklige Form aufweist, kann von einem Quader abweichen und beispielsweise ein Ellipsoid sein.

Ein bevorzugter Abstand zwischen zwei benachbarten Leuchten einer Reihe beträgt 60 bis 65 cm. Ein bevorzugter Abstand der Reihen untereinander beträgt 1,2 bis 1,3 m.

Soll nun innerhalb des Raums 1 die Bestrahlung im Rahmen der Photodynamischen Therapie durchgeführt werden, wird der Patient, nachdem ein zu bestrahlenden behandelndes Hautareal zuvor mit dem Wirkstoff ALA zur Bildung von PPIX in kranken Hautzellen eingecremt worden ist, so in dem Raum gesetzt oder gelegt, dass sich das Hautareal in dem Teilbereich 30 befindet. Durch das Licht der Leuchten 10, 20 (und weiterer Leuchten) bei den oben angegebenen Beleuchtungsstärken wird die gewünschten Reaktionen zur Bildung des Singulettsauerstoffs in Gang gesetzt. Die Konzentration von PPIX und die Konzentration des Singulettsauerstoffes erreichen dabei aber nicht bei weitem die Werte wie bei der konventionellen PDT.

### Bezugszeichenliste

- 1: Raum
- 2: Boden
- 3: Decke
- 4: Seitenwand
- 5: Seitenwand
- 6: Zwischendecke
- 10: Erste Leuchte
- 11: Reflektor
- 12: Leuchtengehäuse
- 13: Leuchtmittel
- 14: Vorschaltgerät
- 15: Lichtstrahl
- 20: Zweite Leuchte
- 21: Reflektor
- 22: Leuchtengehäuse
- 23: Leuchtmittel
- 24: Vorschaltgerät
- 25: Lichtstrahl

## Patentansprüche

1. Raum (1) zur Durchführung der Photodynamischen Therapie (PDT) zur Behandlung von Hautkrankheiten, insbesondere zur Behandlung oberflächlicher Hauttumore wie aktinische Keratosen, flache Basaliome und initiale Karzinome, wobei ein Wirkstoff auf ein zu behandelndes Hautareal zur Bildung von Porphyrinen aufgetragen wird und eine Bestrahlung mit Licht durchgeführt wird, um auf der Basis der Porphyrine Singulettsauerstoff zur Zerstörung kranker Hautzellen zu bilden,
wobei der Raum (1) Seitenwände (4, 5), eine Decke (3) und einen Boden
(2) sowie wenigstens zwei in einer Zwischendecke des Raumes fest installierte Leuchten (10, 20) aufweist, die wenigstens 50 cm voneinander beabstandet sind und zumindest einen Teilbereich (30) des Raums ausleuchten, wobei jede Leuchte einen Reflektor aufweist, der das Licht bündelt und/oder in bestimmter Weise in eine Richtung leitet, so dass sich das Licht mehrerer Leuchten überlagert, so dass an jedem Ort im Teilbereich (30) eine Beleuchtungsstärke von mindestens 8.000 Lux gegeben ist, wobei der Teilbereich eine horizontale Ausdehnung von mindestens 1 m² und eine vertikale Höhe von mindestens 40 cm aufweist, und wobei innerhalb des Teilbereichs (30) die Beleuchtungsstärke nicht mehr als 50 % vom Maximalwert im Teilbereich (30) **gekennzeichnet dadurch, dass**:
die wenigstens zwei Leuchten bündig in der Zwischendecke installiert sind;
ein Abstand zwischen dem Boden und der
Zwischendecke 230 bis 350 cm und ein Abstand zwischen dem Boden (2) und dem Teilbereich (30) 40 bis 120 cm betragen und
die Leuchten jeweils ein Leuchtmittel aufweisen, das im Wellenlängenbereich von 560 bis 660 nm kontinuierlich Licht emittiert und in diesem Bereich 75 Prozent seiner Gesamtstrahlung abgibt.

2. Raum (1) nach Anspruch 1, **dadurch gekennzeichnet, dass** an jedem Ort im Teilbereich (30) die Beleuchtungsstärke nicht größer als 30.000 Lux ist.

3. Raum (1) nach einem der Ansprüche 1 oder 2, **dadurch gekennzeichnet dass** die Leuchten (10, 20) in zwei oder mehr parallelen Reihen angeordnet sind.

4. Raum(1) nach einem der Ansprüche 1 bis 1, **dadurch gekennzeichnet, dass** die Leuchten (10, 20) asymmetrische Reflektoren (11, 21) aufweisen.

5. Raum nach einem der Ansprüche 1 bis 4, **dadurch gekennzeichnet, dass** eine Kühlvorrichtung für die Leuchten (10, 20) vorgesehen ist.

## Claims

1. Space (1) for conducting photodynamic therapy (PDT) for the treatment of skin ailments, in particular for the treatment of superficial skin tumours such as actinic keratosis, flat basaliomas and initial carcinomas, wherein an active agent is applied to a skin area to be treated for the formation of porphyrins and a radiation with light is carried out, in order on the basis of the porphyrins to form singlet oxygen for the destruction of disease skin cells,
wherein the space (1) has side walls (4, 5), a ceiling (3) and a floor (2) as well as at least two lamps (10, 20) permanently installed in an intermediate ceiling of the space, which lamps are spaced at least 50 cm from one another and illuminate at least a partial area (30) of the space, wherein each lamp has a reflector which concentrates the light and/or leads it in a specific manner in a direction such that the light of several lamps overlaps such that there is at every location in the partial area (30) an illumination intensity of at least 8,000 Lux, wherein the partial area has a horizontal extent at least 1 m² and a vertical height of at least 40 cm, and wherein within the partial area (30) the illumination intensity is not more than 50% from the maximum value in the partial area (30), **characterised in that**: the at least two lamps are installed flush in the intermediate ceiling; a distance between the floor and the intermediate ceiling is 230 to 350 cm and a distance between the floor (2) and the partial area (30) is 40 to 120 cm and the lamps have in each case a light source which emits continuous light in the wavelength range from 560 to 660 nm and in this range emits 75 per cent of its total radiation.

2. Space (1) according to claim 1, **characterised in that** at each location in the partial area (30) the illumination intensity is not greater than 30,000 Lux.

3. Space (1) according to any of claims 1 or 2, **characterised in that** the lamps (10, 20) are disposed in two or more parallel rows.

4. Space (1) according to any of claims 1 to 3, **characterised in that** the lamps (10, 20) have asymmetric reflectors (11, 21).

5. Space according to any of claims 1 to 4, **characterised in that** a cooling device is provided for the lamps (10, 20).

## Revendications

1. Pièce (1) de mise en œuvre de la thérapie photodynamique (PDT) pour le traitement de maladies de la peau, en particulier pour le traitement de tumeurs cutanées superficielles telles que kératoses actiniques, basaliomes plats et carcinomes initiaux, dans laquelle un principe actif est appliqué sur une zone de la peau à traiter pour la formation de porphyrines et une irradiation avec de la lumière est effectuée pour former, sur la base des porphyrines, de l'oxygène singulet pour la destruction des cellules cutanées malades, dans laquelle la pièce (1) présente des parois latérales (4, 5), un plafond (3) et un plancher (2) ainsi qu'au moins deux luminaires (10, 20) installés fixement dans un plafond intermédiaire de la pièce, qui sont espacés l'un de l'autre d'au moins 50 cm et éclairent au moins une zone partielle (30) de la pièce, dans laquelle chaque luminaire présente un réflecteur, qui concentre et/ou conduit la lumière de manière déterminée dans une direction, de sorte que la lumière de plusieurs luminaires se superpose, de sorte qu'une intensité d'éclairage d'au moins 8 000 lux est distribuée à chaque endroit de la zone partielle (30), dans laquelle la zone partielle présente une étendue horizontale d'au moins 1 m² et une hauteur verticale d'au moins 40 cm, et dans laquelle à l'intérieur de la zone partielle (30), l'intensité d'éclairage n'est pas supérieure à 50 % de la valeur maximum dans la zone partielle (30), **caractérisée en ce que** :
les au moins deux luminaires sont installés à fleur dans le plafond intermédiaire ;
une distance entre le plancher et le plafond intermédiaire est de 230 à 350 cm et une distance entre le sol (2) et la zone partielle (30) est de 40 à 120 cm et
les luminaires présentent respectivement un moyen lumineux, qui émet de la lumière en continu dans la plage de longueur d'onde de 560 à 660 nm et distribue dans cette plage 75 pour cent de son rayonnement total.

2. Pièce (1) selon la revendication 1, **caractérisée en ce qu'**à chaque endroit de la zone partielle (30), l'intensité d'éclairage n'est pas supérieure à 30 000 lux.

3. Pièce (1) selon l'une quelconque des revendications 1 ou 2, **caractérisée en ce que** les luminaires (10, 20) sont agencés dans deux rangées parallèles ou plus.

4. Pièce (1) selon l'une quelconque des revendications 1 à 3, **caractérisée en ce que** les luminaires (10, 20) présentent des réflecteurs asymétriques (11, 21).

5. Pièce selon l'une quelconque des revendications 1 à 4, **caractérisée en ce qu'**un dispositif de refroidissement est prévu pour les luminaires (10, 20).
